Europäisches Patentamt

European Patent Office

Office européen des brevets

(11) Publication number: **0 219 295 B1**

# EUROPEAN PATENT SPECIFICATION

(19)

(12)

(45) Date of publication of patent specification: **19.01.94**

(21) Application number: **86307707.9**

(22) Date of filing: **06.10.86**

(51) Int. Cl.⁵: **C07K 3/26**, //A61K37/36, A61L2/02

(54) **Process for producing human growth hormone.**

(30) Priority: **07.10.85 JP 223060/85**

(43) Date of publication of application:
**22.04.87 Bulletin 87/17**

(45) Publication of the grant of the patent:
**19.01.94 Bulletin 94/03**

(84) Designated Contracting States:
**CH DE ES FR GB IT LI**

(56) References cited:
**EP-A- 0 085 220**

(73) Proprietor: **NIHON CHEMICAL RESEARCH KABUSHIKI KAISHA also known as JCR PHARMACEUTICALS CO., LTD**
**4-20, Mikagehommachi 3-chome**
**Higashinada-ku**
**Kobe Hyogo 658(JP)**

(72) Inventor: **Hiratani, Hajime**
**705-3, Tottori**
**Hannan-cho**
**Sennan-gun Osaka 599-02(JP)**
Inventor: **Tateishi, Jun**
**5-6-611, Fukuhama 2-chome**
**Chuo-ku Fukuoka 810(JP)**
Inventor: **Kitamoto, Tetsuyuki**
**36 Chaperu Saido Apaato**
**95-1, Goshogadani**
**Chuo-ku Fukuoka 810(JP)**

(74) Representative: **Burford, Anthony Frederick et al**
**W.H. Beck, Greener & Co.**
**7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing human growth hormone. More particularly, the invention relates to a process for producing human growth hormone, which comprises purifying human growth hormone extracted from human pituitaries in improved yield and to such a high extent that there may be eliminated any possibility of contamination with harmful viruses.

In recent years, it has been reported that a disease, assumed to be Creutzfeldt-Jacob disease (hereinafter referred to briefly as "CJD"), occurred in eight patients with pituitary dwarfism in the United States of America and one such patient in the United Kingdom who had been given preparations of human growth hormone produced from human pituitaries employed as a starting material (Scrip No. 995, p. 25, 1985; The Lancet, August 3, p. 244, 1985).

The causative factor of said CJD has not yet been identified, but the slow virus is considered to cause the disease, with the incidence being regarded as about one per million persons.

The pathologic characteristic of CJD is that there are observed spongiform deformity in the cerebral cortex and degeneration in various nervous systems.

Using brain homogenates of mice having developed CJD, inactivation of the virus was attempted by means of different methods by J. Tateishi et al., Department of Neuropathology, Faculty of medicine, Kyushu University, Japan, [Annals of Neurology, 7 (4), 890-891 (1980); Gazette of Medical Society of Japan (Nippon Ishikai Zasshi), 84 (3), 275-282 (1980).

According to the above literature, mouse brain homogenate containing the virus is not inactivated by heat treatment (at 60°C for 30 minutes; at 80°C for 30 minutes; at 100°C for 10 minutes), ultraviolet irradiation, one-year treatment with formalin (10 %) , one-week treatment with glutaraldehyde ( 2 %), one-year treatment with sodium hypochlorite (1 %), one-year treatment with iodine (3 %), one-year treatment with aqueous hydrogen peroxide (30 %), 15-hour treatment with potassium permanganate (2 mM), or 15-hour treatment with 95 % ethanol.

However, it was found that only filtration treatment with a membrane filter of a 0.025$\mu$m pore size and extraction with chloroform :alcohol (3:1) mixture were able to permit removal of the CJD virus, whereas filtration treatment with a membrane filter of an increased pore size as large as 0.10$\mu$m failed to eliminate the CJD virus.

In view of the fact that tile CJD virus is considered to exist more rarely in the human pituitary of a patient afflicted with CJD than in his cerebral cortex, with the incidence of CJD being regarded as one per million persons, the possibility of contamination with the CJD virus of human growth hormone produced from human pituitaries used as a starting material is very small but cannot be entirely ruled out.

The present inventors, with a specific view to removing tic CJD virus suspected of contamination of human growth hormone in its production process, attempted to incorporate the step of filtration through the above-described 0.025 $\mu$m membrane filter into the production process.

In the filtration through a membrane filter, nevertheless, it was found that particularly membrane filters of a decreased pore size adsorb greatly human growth hormone itself, thus reducing markedly the yield of human growth hormone.

Consequently, the present inventors conducted extensive investigation into a method permitting the removal of the CJD virus without lowering the yield of human growth hormone, and as a result, found that filtration of a human growth hormone solution using a membrane filter subjected to pretreatment with a water-soluble high molecular substance such as serum albumin can prevent adsorption of the hormone onto the membrane filter and realize an enhanced yield, as high as 90 to 100 %.

This invention is based on the above new finding and is directed to a process for producing human growth hormone which comprises filtering the solution of human growth hormone through a membrane filter of 0.025 - 0.05 $\mu$m in pore size, said membrane filter being previously treated with a solution of a water-soluble high molecular substance which substantially does not hinder said solution of human growth hormone from passing through said filter.

In this invention, the phrase "substantially does not hinder said solution of human growth hormone from passing through the filter" means that at least the major activity of the hormone is able to pass through the filter. Human growth hormone is a very expensive material, therefore, it is desirable to recover more than 80 % of the hormone activity in the filtrate.

As the water-soluble high molecular substance, there may be exemplified human serum albumin, polyvinylpyrrolidone, polyethyleneglycol, polyethylene-polypropyleneglycol, gelatin or modified gelatin such as Poligeline (Höchst, Japan) or polyoxyethylenesorbitan esters of fatty acids such as polysorbate 80 (Tween 20; Nakarai Kagaku Co., Japan) or polysorbate 20 (Tween 80; ibid.)

The pretreatment of the membrane filter can be performed conveniently by filtering an aqueous solution of the high molecular substance through the filter, whereby the substance is adsorbed onto the filter. The adsorption may also be performed by impregnating the filter with an aqueous solution of the substance.

The concentration of the high molecular substance in the solution can be selected from wide range provided the purpose of the pretreatment is not obstructed; however, a concentration from 2 to 0,2 %, usually is sufficient.

The membrane filters having a pore size of 0.025 to 0.05$\mu$m are available as commercially marketed articles supplied by Millipore Co. of Japan, Sartorius Co. of West Germany and Nuclepore Co. of U.S.A.

The membrane filter which has been pretreated with a water-soluble high molecular substance is washed with water to remove excess of the substance.

With use of the thus-prepared filter, a human growth hormone solution is subjected to filtration, whereby a human growth hormone solution can be prepared in the form an aqueous solution, for example a physiological saline solution. The purity and concentration of human growth hormone in the said solution can be selected over such a range as may not impede the filtration through the specifically determined membrane filter, but needless to say, an enhanced degree of purity of the hormone facilitates the operation.

By the above procedure, human growth hormone solution can be filtered through a membrane filter in improved yield.

In this invention, the pretreatment of a membrane filter with a water-soluble substance prevents the adsorption of human growth hormone onto the filter in the subsequent filtration of a human growth hormone solution, thereby rendering it possible to allow the human growth hormone to pass through the membrane filter efficiently in improved yield, which enables the efficient removal of an CJD virus present.

The Reference Example and Examples following illustrate this invention in more detail, but the invention is not limited by them.

Reference Example 1

1. Experimental materials

Membrane filters produced by Millipore Co. of Japan:

| Pore size | Catalogue No. |
|-----------|---------------|
| 0.01 $\mu$m | (VSWP02500) |
| 0.050 $\mu$m | (VMWP02500) |
| 0.10 $\mu$m | (VCWP02500) |

Membrane filters produced by Sartorius Co. of West Germany:

| Pore size | Catalogue No. |
|-----------|---------------|
| 0.015 $\mu$m | (11318) |
| 0.05 $\mu$m | (11328) |
| 0.10 $\mu$m | (11358) |

Mmebrane filters produced by Nuclepore Co. of U.S.A.A:

| Pore size | Catalogue No. |
|-----------|---------------|
| 0.015 $\mu$m | (110601) |
| 0.03 $\mu$m | (110602) |
| 0.05 $\mu$m | (110603) |
| 0.08 $\mu$m | (110604) |

2. Experimental method

The following experiment was carried out with 80 mℓ (0.60 IU/mℓ) of a human growth hormone solution (a physiological saline solution):

Using a 10-ml disposable syringe, 7 ml of the human growth hormone solution for each filter was filtered through a filter of 25 cm diameter fixed onto a filter holder.

Throughout the experiment, each filter was autoclaved in advance (at 120°C for 30 minutes), followed by passing 5 ml of physiological saline and the test solution (human growth hormone solution), successively, through each membrane.

The human growth hormone was quantitatively determined by radioimmunoassay (RIA Kit manufactured by Dalnabot Co.). 3. Experimental results

The results are shown in Table 1.

Table 1:

| Membrane filter | Recovery (%) of HGH* activity in filtrate |
|---|---|
| **Produced by Millipore Co.** | |
| 0.025 µm | 62.4 |
| 0.05 µm | 69.0 |
| 0.10 µm | 68.3 |
| **Produced by Sartorius Co.** | |
| 0.01 µm | Difficult to be filtered |
| 0.05 µm | 72.9 |
| 0.10 µm | 74.6 |
| Produced by Nuclpore Co. | |
| 0.015 µm | Difficult to be filtered |
| 0.03 µm | 87.1 |
| 0.05 µm | 101.7 |
| 0.08 µm | 99.6 |
| Solution prior to filtration through a membrane filter: | 100.0 |

Note: *   HGH stands for human growth hormone (the same is to be applied hereinafter).

Example 1

1. Experimental materials

Membrane filters produced by Millipore Co.:

| Pore size | | Catalogue No. |
|---|---|---|
| 0.025 | μm | (VSWP 02500) |
| 0.05 | μm | (VMWP 02500) |
| 0.10 | μm | (VCWP 02500) |

Membrane filters produced by Sartorius Co.:

| Pore size | | Catalogue No. |
|---|---|---|
| 0.05 | μm | (11328) |
| 0.10 | μm | (11358) |

20 % human serum albumin (Chemical & Serum Therapy Research Laboratories (a juridical person), Lot. A 325)

2. Experimental method

As a test solution, there was used 40 ml of the human growth hormone solution. Each filter was fixed onto a filter holder and then autoclaved (at 120°C for 30 minutes), and the filtration procedure was carried out using 5 ml of 0.2 % human serum albumin solution (prepared by dilution with physiological saline) to perform the coating of the membrane filters.

Subsequently, the filters were washed twice with physiological saline (5 ml x 2) to remove excessive human serum albumin, and then the filtration experiment was carried out with 7 ml of the human growth hormone solution.

The human growth hormone was quantitatively determined by radioimmunoassay (RIA Kit manufactured by Dynabott Co.).

The filtrate of the human growth hormone solution was investigated for contamination of the human growth hromone with human serum albumin by means of SDS-polyacrylamide slab gel electrophoresis. 3. Experimental results

The results are shown in Table 2.

Table 2:

| Membrane filter | Recovery (%) of HGH*activity in filtrate |
|---|---|
| Produced by Millipore Co. | |
| 0.025 μm | 96.3 |
| 0.05 μm | 97.0 |
| 0.10 μm | 99.8 |
| Produced by Sartorius Co. | |
| 0.05 μm | 96.0 |
| 0.10 μm | 97.4 |
| Solution prior to filtration through a membrane filter: | 100.0 |

Analysis by SDS-polyacrylamide slab gel electrophoresis indicated that there was no contamination of the HGH solution (filtrate) with human serum albumin.

Example 2

1. Experimetal materials:

(1) Membrane filter (0.025$\mu$m, Cat. No. VSWP 02500) [Millipore Co. Ltd., Japan]
(2) Human growth hormone ["Crorum", Cerono Co. Ltd., Switzerland]
(3) Human serum albumin [Chemo- & Serum-therapy Research Foundation, Japan]
(4) Dextran (M. W. 5000-7000) [Nakarai Kagaku Co. Ltd., Japan]
(5) PVP-25 (Polyvinylpyrrolidone 25) [Nakarai Kagaku Co. Ltd., Japan]
(6) L-arginine hydrochloride [Nakarai Kagaku Co. Ltd., Japan]
(7) PEG-4000 (Polyethyleneglycol 4000) [Wako Junyaku Co. Ltd., Japan]
(8) PEG-5000 (Polyethylene-polypropyleneglycol 5000) [Nishio Kogyo Co. Ltd., Japan]
(9) Polygeline (Modified gelatin) [Höchst, Japan]
(10) Human Immune globulin [Globelin-I; Nippon Seiyaku Co. Ltd., Japan]
(11) Gelatin (pure powder) [Nakarai Kagaku Co. Ltd., Japan]
(12) MDS (Methyl dextran sulfate) [Kowa Co. Ltd., Japan]
(13) Tween 20 (Polyoxyethylenesorbitan monolaurate) [Nakarai Kagaku Co. Ltd., Japan]
(14) Tween 80 (Polyoxyethylenesorbitan monooleate) [Nakarai Kagaku Co. Ltd., Japan]

2. Experimental method:

Membrane filters were fitted onto respective filter holders of 25mm diameter and heated at 120°C for 30 minutes in an autoclave.

0.5% aqueous solutions of each of materials (3) to (14) were prepared. Each of the membrane filters was pre-treated by filtering 0.5 ml of a respective solution through the filter.

Subsequently, each membrane filter was washed two times with physiological saline (5ml x 2) to remove excess of the coating material, and then filtration tests were carried out employing each 7 ml of human growth hormone solution (0.6 IU/ml).

The activity of human growth hormone was assayed with a radioimmunoassay kit (Dainabot Co. Ltd., Japan). 3. Results:

The test results are shown in the following Table:

| Pre-treating solutions | Recovery rate of HCG activity in filtrate (Activity before filtration = 100) |
|---|---|
| Physiological saline | 52.5 |
| 0.5 % solution of human serum albumin | 98.0 |
| 0.5 % solution of dextran | 56.1 |
| 0.5 % solution of PVP-25 | 103.0 |
| 0.5 % solution of arginine | 54.5 |
| 0.5 % solution of PEG-4000 | 51.0 |
| 0.5 % solution of PEG-5000 | 74.3 |
| 0.5 % solution of Polygeline | 88.6 |
| 0.5 % solution of human immune globulin | ---* |
| 0.5 % solution of MDS | 40.1 |
| 0.5 % solution of Tween 20 | 97.0 |
| 0.5 % solution of Tween 80 | 94.1 |
| 0.5 % solution of gelatin | 93.5 |

* Pre-treatment was impossible because of difficulty in filtration.

6

**Claims**

1. A process for producing human growth hormone which comprises filtering a solution of human growth hormone through a membrane filter of 0.025 - 0.05 $\mu$m pore size, characterised in that said membrane filter has previously been treated with a solution of a water-soluble high molecular substance selected from human serum albumin, polyvinylpyrrolidone, polyethyleneglycol, polyethylene-polypropylene-glycol, modified or unmodified gelatin and polyoxyethylenesorbitan esters of fatty acids, which treatment substantially does not hinder said solution of human growth hormone from passing through said filter.

2. A process as claimed in Claim 1 including the step of treating said membrane with the solution of said water-soluble high molecular substance.

3. A process as claimed in Claim 1 or Claim 2, wherein said high molecular weight substance is human serum albumin.

4. A process as claimed in Claim 1 or Claim 2, wherein said high molecular weight substance is polyvinylpyrrolidone 25.

5. A process as claimed in Claim 1 or Claim 2, wherein said high molecular weight substance is polyoxyethylenesorbitan monooleate.

6. A process as claimed in Claim 1 or Claim 2, wherein said high molecular weight substance is polyoxyethylenesorbitan monolaurate.

7. A process as claimed in Claim 1 or Claim 2, wherein said high molecular weight substance is modified or unmodified gelatin.

8. A process as claimed in any of the preceding claims, wherein the solution of a water-soluble high molecular substance is an aqueous solution containing the substance in a concentration from 2 to 0.2%.

9. A process as claimed in any one of the preceding claims, wherein the said treatment of the membrane filter is carried out by passing the solution of a water-soluble high molecular substance through the filter.

**Patentansprüche**

1. Verfahren zur Herstellung des menschlichen Wachstumshormons, welches das Filtrieren einer Lösung des menschlichen Wachstumshormons durch einen Membranfilter mit einer Porengröße von 0,025 - 0,05 $\mu$m umfaßt, dadurch gekennzeichnet, daß der Membranfilter zuvor mit einer Lösung einer aus humanem Serumalbumin, Polyvinylpyrrolidon, Polyethylenglykol, Polyethylenpolypropylenglykol, modifizierter oder nicht-modifizierter Gelatine und Polyoxyethylensorbitanester von Fettsäuren ausgewählten wasserlöslichen hochmolekularen Substanz behandelt worden ist, wobei die Lösung des menschlichen Wachstumshormons durch die Behandlung nicht in wesentlicher Weise daran gehindert wird, den Filter zu passieren.

2. Verfahren nach Anspruch 1, welches den Schritt der Behandlung der Membran mit der Lösung der wasserlöslichen hochmolekularen Substanz einschließt.

3. Verfahren nach den Ansprüchen 1 oder 2, bei dem die hochmolekulare Substanz humanes Serumalbumin ist.

4. Verfahren nach den Ansprüchen 1 oder 2, bei dem die hochmolekulare Substanz Polyvinylpyrrolidon 25 ist.

5. Verfahren nach den Ansprüchen 1 oder 2, bei dem die hochmolekulare Substanz Polyoxyethylensorbitanmonooleat ist.

**6.** Verfahren nach den Ansprüchen 1 oder 2, bei dem die hochmolekulare Substanz Polyoxyethylensorbitanmonolaurat ist.

**7.** Verfahren nach den Ansprüchen 1 oder 2, bei dem die hochmolekulare Substanz modifizierte oder nicht-modifizierte Gelatine ist.

**8.** Verfahren nach einem der vorhergehenden Ansprüche, bei dem die Lösung einer wasserlöslichen hochmolekularen Substanz eine wäßrige Lösung ist, die die Substanz in einer Konzentration von 2 bis 0,2 % enthält.

**9.** Verfahren nach einem der vorhergehenden Ansprüche, bei dem die Behandlung des Membranfilters erfolgt, indem man die Lösung einer wasserlöslichen hochmolekularen Substanz den Filter passieren läßt.

**Revendications**

**1.** Procédé de production de l'hormone de croissance humaine qui consiste à filtrer une solution d'hormone de croissance humaine à travers une membrane de filtration ayant une taille de pores de 0,025 à 0,05 $\mu$m, caractérisé en ce que ladite membrane de filtration a préalablement été traitée par une solution d'une substance de haut poids moléculaire hydrosoluble choisie entre la sérumalbumine humaine, la polyvinylpyrrolidone, la polyéthylèneglycol, le polyéthylènepolypropylèneglycol, la gélatine modifiée ou non modifiée et les esters de polyoxyéthylènesorbitanne d'acides gras, traitement qui substantiellement n'empêche pas ladite solution d'hormone de croissance humaine de passer à travers ladite membrane de filtration.

**2.** Procédé selon la revendication 1, comprenant l'étape de traitement de ladite membrane par la solution de ladite substance de haut poids moléculaire hydrosoluble.

**3.** Procédé selon la revendication 1 ou la revendication 2, dans lequel ladite substance de haut poids moléculaire est la sérumalbumine humaine.

**4.** Procédé selon la revendication 1 ou la revendication 2, dans lequel ladite substance de haut poids moléculaire est la polyvinylpyrrolidone 25.

**5.** Procédé selon la revendication 1 ou la revendication 2, dans lequel ladite substance de haut poids moléculaire est le monooléate de polyoxyéthylènesorbitanne.

**6.** Procédé selon la revendication 1 ou la revendication 2, dans lequel ladite substance de haut poids moléculaire est le monolaurate de polyoxyéthylènesorbitanne.

**7.** Procédé selon la revendication 1 ou la revendication 2, dans lequel ladite substance de haut poids moléculaire est la gélatine modifiée ou non modifiée.

**8.** Procédé selon l'une quelconque des revendications précédentes, dans lequel la solution d'une substance de haut poids moléculaire hydrosoluble est une solution aqueuse contenant la substance à une concentration de 2 à 0,2%.

**9.** Procédé selon l'une quelconque des revendications précédentes, dans lequel ledit traitement de la membrane de filtration est effectué en faisant passer la solution d'une substance de haut poids moléculaire hydrosoluble à travers la membrane de filtration.